# EUROPEAN PATENT APPLICATION

(11) **EP 1 647 186 A1**
(43) Date of publication of application: **19.04.2006**
(21) Application number: 05022287.6
(22) Date of filing: 12.10.2005
(51) Int. Cl.: A01N 1/02, C12N 1/04

(54) **Cell-preservation liquid**

(30) Priority: 12.10.2004 JP 2004297532; 28.01.2005 JP 2005022170
(71) Applicant: NIPRO CORPORATION, Osaka-shi, Osaka 531-8510 (JP)
(72) Inventor: Taguchi, Atsushi, Osaka-shi Osaka, 531-8510 (JP); Sato, Takeshi c/o Cell Science & Techn. Inst. Inc., Sendai-shi Miyagi, 989-3204 (JP); Yabe, Noritsugu c/o Cell Science & Tech. Inst. Inc, Sendai-shi Miyagi, 989-3204 (JP)
(74) Representative: Albrecht, Thomas

(57) **Abstract**

A cell-preservation liquid containing at least a saccharide, sodium ion, and potassium ion, which is characterized in that the molar concentration of the contained sodium ion is equal to or more than that of the potassium ion is provided. The preservation liquid enables not only cryopreservation but also cold preservation, and an easy cell-preservation method may be provided. Moreover, when the cell-preservation liquid of the present invention is used, cell culture may be performed by inoculating cells in a medium with the cells suspended in the preservation liquid without a washing step or the like, so that the cells may be easily cultured.

## Description

### Field of the Invention

The present invention relates to: a cell-preservation liquid containing at least a saccharide, sodium ion, and potassium ion, in which the concentration of the contained sodium ion is equal to or more than that of the potassium ion; and a cell-preservation method using the preservation liquid.

### Background of the invention

A living cell has physiological functions and biological activities and has utility in many fields. However, when the cell is allowed to stand under a high-temperature condition, the cell physiological functions and biological activities are decreased or lost due to metabolism and degeneration. Accordingly, cells are required to be cryopreserved. Conventionally, for cell cryopreservation, DMSO (dimethyl sulfoxide), ethylene glycol, glycerol, or the like has been used as a cell membrane permeable cryopreserving agent, while polyvinylpyrrolidone or the like has been used as a cell membrane nonpermeable cryopreserving agent. It is disclosed that when a cell-preservation liquid containing purified albumin and DMSO is used, cells may be preserved at -80°C or, if for a short period of time, at about -4°C (JP 2002-233356 A).

However, those cryopreserving agents have a problem with toxicity to cells when they exist in high concentrations. In the case of cell culture, the above-described cryopreserving agents inhibit cell proliferation, so that they are required to be removed in advance from the cells, which is cumbersome. Cryopreservation is performed under an extremely low temperature, for example, -80°C, which has a problem that an expensive technical freezing apparatus is required. A freezing/thawing process inevitably causes severe damage to cells, resulting in a decrease in the survival rate. Some cell species cannot be cryopreserved.

As cell/tissue-preservation liquids for cryopreservation that contain no substance such as DMSO capable of exhibiting toxicity, disclosed are a preservation liquid containing levan or levanoligosaccharide that is a polysaccharide (JP 9-255501 A), a preservation liquid containing 1-kestose that is one kind of fracoligosaccharides (JP 5-38284 A), and the like.

Meanwhile, there are reports that a composition in which trehalose, a matrix protein filler, and a monosaccharide are contained in a buffer (JP 2003-505024 A) and a preservation liquid containing polyphenol that has an antioxidant effect (International Patent WO02/01952 A) are useful for preserving cells or organs at room temperature. The former is used for a preservation method including mixing cells in the composition and drying the mixture. In addition, when cells are suspended in a solution to which a chelating agent or the like is added to an alcohol capable of being blended in water, the cells may be preserved at about 37°C for about 3 weeks (JP 2002-168858 A).

Conventionally used cell/tissue-preservation liquids other than the above-described solutions include Euro-Collins solution (Wahlberg, J. A., et al., Transplantation, 43, pp. 5-8, 1987) and UW solution (University of Wisconsin solution) (Squifflet, J. P., et al., Transplant Proc., 13, 693, 1981). However those solutions have problems of insufficient effects for maintaining cell physiological functions and of pharmaceutical instability. For solving those problems, disclosed are ET-Kyoto solution (JP 6-40801 A) and a solution to which epigallocatechin gallate that is a green tea catechin is added as an active ingredient (JP 2003-267801 A).

### Summary of the Invention

An object of the present invention is a cell-preservation liquid having an improved cell-preservation ability, which enables not only cryopreservation but also cold preservation and enables cell culture or the like even in the presence of the preservation liquid without a requirement of washing to remove the preservation liquid.

To solve the above-described problems, the inventors of the present invention have made extensive studies. As a result, they have focused attention on the fact that a cell-preservation liquid containing at least a saccharide, sodium ion, and potassium ion, in which the molar concentration of the contained sodium ion is equal to or more than that of the potassium ion has more improved cell-preservation ability than a conventional cell-preservation liquid, e.g., Euro-Collins solution. Thus the cell-preservation liquid of the present invention has been accomplished.

That is, the present invention provides the following:
(1) A cell-preservation liquid containing at least a saccharide, sodium ion, and potassium ion, wherein the molar concentration of the contained sodium ion is equal to or more than that of the potassium ion.
(2) The cell-preservation liquid according to the above item 1, wherein the molar ratio of the potassium ion and the sodium ion is 1:1 to 1:85.
(3) The cell-preservation liquid according to the above item 1, wherein the saccharide content is 0.5 to 150 mM.
(4) The cell-preservation liquid according to the above item 1, wherein the saccharide is any one or a plurality of saccharides selected from the group consisting of glucose, maltose, mannitol, sucrose, and trehalose.
(5) The cell-preservation liquid according to the above item 1, wherein the saccharide is glucose and/or maltose.
(6) The cell-preservation liquid according to the above item 1, which further contains bicarbonate ion and/or carbonate ion.
(7) The cell-preservation liquid according to the above item 6, wherein the bicarbonate ion and/or carbonate ion content is 1 to 75 mM.
(8) The cell-preservation liquid according to the above item 1, which further comprises any one kind or a plurality of kinds of ions selected from the group consisting of calcium ion, magnesium ion, chloride ion, phosphate ion, sulfate ion, and lactate ion.
(9) The cell-preservation liquid according to the above item 8, which contains the following composition in 1,000 mL of a solution.

| | |
|---|---|
| Saccharide | 0.5 to 150 mM |
| Sodium ion | 20 to 260 mM |
| Potassium ion | 3 to 125 mM |
| Bicarbonate ion and/or carbonate ion | 1 to 75 mM |
| Calcium ion | 1 to 4 mM |
| Magnesium ion | 0 to 1 mM |
| Chloride ion | 75 to 245 mM |
| Phosphate ion | 0.5 to 65 mM |
| Sulfate ion | 0 to 1 mM |
| Lactate ion | 0 to 20 mM |

(10) A method for preparing the cell-preservation liquid according to the above item 1, which is characterized in that a sodium salt, a potassium salt and a saccharide are mixed with a solvent selected from the group consisting of a dehydration supplementary liquid and pure water such that the molar concentration of the contained sodium ion is equal to or more than that of the potassium ion.
(11) A method for preparing the cell-preservation liquid according to the above item 10, which is characterized in that Ringer's solution as defined in the Japanese Pharmacopoeia, sodium bicarbonate injection as defined in the Japanese Pharmacopoeia, and a commercially available dehydration supplementary liquid are mixed at a volume ratio of 80:3:20 to 50.
(12) The method for preparing the cell-preservation liquid according to the above item 10, wherein the commercially available dehydration supplementary liquid is selected from the injection solutions shown in 1) and 2) below:
1) Injection solution containing the components described below in 1,000 mL of solution;

| | |
|---|---|
| Sodium chloride | 1.92 g |
| Potassium chloride | 1.00 g |
| Sodium lactate | 2.80 g |
| Magnesium chloride | 0.10 g |
| Monosodium phosphate | 0.14 g |
| Dipotassium phosphate | 1.00 g |
| Glucose | 23.5 g |

2) Injection solution containing the composition described below;

| | |
|---|---|
| Sodium chloride | 0.270 w/v% |
| Potassium chloride | 0.149 w/v% |
| Sodium lactate | 0.224 w/v% |
| Sodium dihydrogenphosphate | 0.031 w/v% |
| Sodium monohydrogenphosphate | 0.287 w/v% |
| Glucose | 3.2 w/v% |
| L-lactic acid as an additive | 8 mEq/L |

(13) A cell-preservation method characterized in that a cell is preserved under a temperature of above freezing to 25 °c using the cell-preservation liquid according to the above items 1 to 9.
(14) The cell-preservation method according to the above item 13, wherein the cell is a lymphocyte.
(15) A cell-culture method including the step of: suspending a cell in the cell-preservation liquid according to the above items 1 to 9; and inoculating the suspension without treatment in a medium to culture the cell.

A cell-preservation liquid containing at least a saccharide, sodium ion, and potassium ion, in which the molar concentration of the contained sodium ion is equal to or more than that of the potassium ion, leads to an increase in a cell survival rate and maintenance of a cell physiological function, so that a cell-preservation liquid capable of maintaining a cell-preservation ability at a high level may be provided. In addition, when the cell-preservation liquid of the present invention is used, a method for preserving cells may be provided not only in the case of cryopreservation but also under cold conditions. Furthermore, when the cell-preservation liquid of the present invention is used, a cell-culture method performed by inoculating and culturing cells in a medium with the cells being suspended in the preservation liquid without a washing step or the like can be provided.

### Brief Description of the Drawings

Fig. 1 is a graph showing variations with time of survival rates in the case of lymphocyte preservation in the cell-preservation liquids of Examples 1 and 16 and Comparative Examples 2 and 3 (Experimental Example 4).
Fig. 2 is a graph showing variations with time of survival rates in the case of lymphocyte preservation in the cell-preservation liquids of Examples 18 to 21 and Comparative Examples 2 and 3 (Experimental Example 4).

### Detailed Description

The cell-preservation liquid of the present invention is characterized by containing at least a saccharide, sodium ion, and potassium ion, in which the molar concentration of the contained sodium ion is equal to or more than that of the potassium ion.

Examples of the saccharide to be used in the present invention include various monosaccharides, disaccharides, trisaccharides, polysaccharides, and sugar alcohol, such as glucose, galactose, fructose, ribose, mannose, trehalose, sucrose, maltose, and mannitol. These saccharides may be used singly or in a combination of two or more saccharides. Of these, glucose, maltose, mannitol, sucrose, and trehalose are preferable in terms of economical efficiency/handiness, and glucose and maltose are more preferable. As the saccharides, commercially available ones may be used. The saccharide content in a cell-preservation liquid is 0.5 to 150 mM, preferably 0.5 to 100 mM, and more preferably 0.5 to 50 mM. When the saccharide content exceeds 150 mM, the cells are sometimes aggregated and turn into masses in preserving cells, and a cumbersome procedure is required for breaking the masses, which is not preferable.

The sodium ion and potassium ion to be used in the present invention may be provided in the preservation liquid by adding a sodium or potassium salt of an organic acid, sodium chloride, or the like. In the case that the molar concentration of the contained sodium ion is higher than that of the potassium ion, the preservation liquid may be an extracellular fluid type preservation liquid and contain the potassium ion and sodium ion at a molar ratio in the range of 1:1 to 1:85, preferably in the range of 1:2 to 1:60, and more preferably in the range of 1:5 to 1:30. Specifically, the sodium ion content is 20 to 260 mM, preferably 50 to 200 mM, and more preferably 100 to 150 mM. In the case that the molar ratio of the potassium ion and sodium ion is in the range satisfying the above-described conditions, the potassium ion content may be from 3 to 125 mM, preferably 3 to 100 mM, and more preferably 3 to 75 mM.

The cell-preservation liquid of the present invention preferably further contains bicarbonate ion and/or carbonate ion. In the present specification, the bicarbonate ion and carbonate ion represent HCO₃⁻ and CO₃²⁻, respectively. These ions are in equilibrium in a solution and may vary depending on a condition such as pH. The bicarbonate ion and/or carbonate ion may be contained in the cell-preservation liquid by adding various bicarbonates and carbonates to the cell-preservation liquid. Examples of such compounds include sodium bicarbonate (which is also referred to as "baking soda"), sodium carbonate, calcium carbonate, calcium bicarbonate, and magnesium carbonate, and preferable is sodium bicarbonate (baking soda) in terms of economical efficiency/handiness. As sodium bicarbonate, a commercially available one may be used. (In the Examples described below, sodium bicarbonate manufactured by Wako Pure Chemical Industries, Ltd. was used). The bicarbonate ion and/or carbonate ion content in the cell-preservation liquid may be from 1 to 75 mM, preferably 10 to 50 mM, or more preferably 20 to 30 mM.

The cell preservation liquid of the present invention prevents swelling or shrinkage of cells and tissues or the like, so that the cell survival rate may be improved. Accordingly, the osmotic pressure of the cell-preservation liquid is regulated to 200 to 500 mOsm/kg, preferably 200 to 400 mOsm/kg, and more preferably 250 to 350 mOsm/kg. The concentrations of all substances such as ions contained in the preservation liquid control the osmotic pressure of the preservation liquid. The osmotic pressure of the preservation liquid may be regulated appropriately by varyirig the contents of the saccharide, bicarbonate ion and/or carbonate ion, and other ions contained in the preservation liquid or by adding a harmless osmotic pressure regulator such as hydroxyethyl starch.
In addition, the preservation liquid of the present invention is regulated to pH 5.0 to 8.5, preferably pH 6.0 to 8.5, and more preferably pH 7.0 to 8.0. The pH value may be regulated by adding an electrolyte such as the above-described sodium or potassium salt of an organic acid.

The cell-preservation liquid of the present invention may further contain ions that may generally be contained in a preservation liquid such as metal ions including calcium ion and magnesium ion, chloride ion, phosphate ion, nitrate ion, sulfate ion, and lactate ion. Those ions may be used singly or in a combination of two or more ions. The concentrations of ions is difficult to be specified without variation because they depend on the osmotic pressure and pH of the preservation liquid, the contents of other substances in the preservation liquid, and the like. However, for example, the concentrations may be selected from: 0.0 to 1 mM or preferably 0.2 to 0.8 mM (magnesium ion); 1 to 4 mM or preferably 1.5 to 3.5 mM (calcium ion); 75 to 245 mM or preferably 100 to 150 mM (chloride ion); 0.5 to 65 mM or preferably 0.5 to 2 mM (phosphate ion); 0 to 1 mM or preferably 0.5 to 1 mM (sulfate ion); and 0 to 20 mM or preferably 0 to 15 mM (lactate ion).

The cell-preservation liquid of the present invention may appropriately contain other ingredients contained in a conventional preservation liquid such as an antibiotic, antimicrobial agent, antioxidant, serum, vitamin, protein, amino acid, pH indicator, or chelating agent.

The cell-preservation liquid of the present invention may be prepared by a method including mixing a dehydration supplementary liquid that is a commercially available infusion preparation with Ringer's solution as defined in the Japanese Pharmacopoeia and sodium bicarbonate solution as defined in the Japanese Pharmacopoeia under an aseptic condition so that required ingredients are contained at required concentrations, which is preferable. The cell-preservation liquid may also be prepared by another method including weighing required amounts of respective required ingredients, mixing them in pure water, and filtering the mixture to eliminate bacteria.
As a dehydration supplementary liquid that is a commercially available infusion preparation, for example, KN-solution series (manufactured by Otsuka Pharmaceutical Co., Ltd.) as an agent that is sold as a multiple electrolyte infusion and/or SORITA™ series and EL-solution series (both manufactured by AJINOMOTO CO., Inc. and AJINOMOTO PHARMA CO., Inc.) as an electrolyte solution for infusion may be used. More specifically, injection solutions having the following compositions 1) or 2) may be used.

1) Injection solution containing the components described below in 1,000 mL of the solution:

| | |
|---|---|
| Sodium chloride | 1.92 g |
| Potassium chloride | 1.00 g |
| Sodium lactate | 2.80 g |
| Magnesium chloride | 0.10 g |
| Monosodium phosphate | 0.14 g |
| Dipotassium phosphate | 1.00 g |
| Glucose | 23.5 g |

2) Injection solution containing the components described below in 1,000 ml of the solution:

| | |
|---|---|
| Sodium chloride | 0.270 w/v% |
| Potassium chloride | 0.149 w/v% |
| Sodium lactate | 0.224 w/v% |
| Sodium dihydrogenphosphate | 0.031 w/v% |
| Sodium monohydrogenphosphate | 0.287 w/v% |
| Glucose | 3.2 w/v% |
| L-lactic acid as an additive | 8 mEq/L |

Ringer's solution as defined in the Japanese Pharmacopoeia, sodium bicarbonate solution in as defined the Japanese Pharmacopoeia, and a commercially available infusion preparation may be mixed at a volume ratio of 80:3:20 to 50. Specifically, the preservation liquid may be prepared by mixing the injection described in 1) above (250 to 300 mL) with Ringer's solution as defined in the Japanese Pharmacopoeia (800 mL) and sodium bicarbonate solution as defined in the Japanese Pharmacopoeia (30 mL). As another method, the preservation liquid may be prepared by mixing the injection described in 2) above (400 to 450 mL) with Ringer's solution as defined in the Japanese Pharmacopoeia (800 mL) and sodium bicarbonate solution as defined in the Japanese Pharmacopoeia (30 mL).

Examples of cells that may be preserved using the cell-preservation liquid of the present invention include various cells isolated from: mammals including human such as lymphocytes, stem cells, skin cells, mucosal cells, hepatic cells, pancreatic islet cells, nerve cells, cartilage cells, endothelial cells, epithelial cells, bone cells, muscular cells, bone marrow cells, and hematopoietic stem cells; animals other than mammals including fish such as sperm, ovum, and fertilized ovum; and insect cells. For example, the preservation liquid may also be used when a human activated lymphocyte is preserved.

The present invention encompasses a cell-preservation method using the above-described cell-preservation liquid. As a preservation method, cells suspended in the cell-preservation liquid may be preserved after freezing as usual or preserved under a cold condition without freezing. The cold condition is a condition where a solution does not freeze and may be a low temperature, which is in the range of preferably 0 to 25°C, more preferably 0 to 10°C, and particularly preferably 4 to 6°C.

The preservation period in using the cell-preservation liquid of the present invention is difficult to specify without variation because it depends on conditions such as cell species and preservation temperature, but it is generally about 8 to 12 days, preferably about 4 to 6 days. As a specific use aspect, for example, the preservation liquid may be used for preservation when activated lymphocytes are transported from a facility for cell culture service to a patient. In the case of such a short-period preservation, the preservation liquid and preservation method of the present invention have an advantage in that cells may be preserved under a cold condition.

In preserving cells, the concentration of the cells to be suspended in the cell-preservation liquid is difficult to specify without variation because it depends on conditions such as cell species, cell size, and preservation period, but it is about 1.0 × 10⁴ to 1.0 x 10⁸ cells/mL, preferably about 1.0 x 10⁵ to 1.0 x 10⁸ cells/mL. Meanwhile, a preservation container to be used may be appropriately selected from known containers with consideration for cell species, preservation temperature, preservation period, application of preserved cells, and the like. dose of γ-rays was 25 kGy.

Moreover, the present invention encompasses a cell-culture method using cells preserved as described above. The preserved cells may be inoculated in a general medium and cultured after separating the cells from the preservation liquid by centrifugation or the like, or a preserved cell suspension in the cell-preservation liquid may be inoculated in a general medium and cultured without treatment.

### [Example 1] Cell-preservation liquid containing glucose and baking soda

A cell-preservation liquid having the following composition was prepared. Each compound was dissolved in pure water 1000ml. The range of osmotic pressure was regulated from 280 to 300 mOsm/kg. The osmotic pressure was measured using OSMOMETER OM 802-D (manufactured by VOGEL).

| | |
|---|---|
| Glucose (Wako Pure Chemical Industries, Ltd.) | 1,000 mg |
| Calcium chloride | 200 mg |
| Potassium chloride | 400 mg |
| Magnesium sulfate | 98 mg |
| Sodium chloride | 6,800 mg |
| Phosphoric acid hydrogen sodium hydrate | 140 mg |
| Carbonic acid hydrogen sodium (baking soda) | 2,000 mg |
| Pure water | |
| Total | 1,000 mL |

The ion composition of this cell-preservation liquid is shown in the following.

| | |
|---|---|
| Glucose | 5.5 mM |
| Sodium ion | 143.8 mM |
| Potassium ion | 5.4 mM |
| Bicarbonate ion and/or carbonate ion | 23.7 mM |
| Calcium ion | 1.8 mM |
| Magnesium ion | 0.8 mM |
| Chloride ion | 130.8 mM |
| Phosphate ion | 1.0 mM |
| Sulfate ion | 0.8 mM |
| Pure water | |
| Total | 1,000 mL |

### [Example 2] Cell-preservation liquid containing maltose and baking soda

A cell-preservation liquid similar to that of Example 1, except that glucose in Example 1 was replaced by maltose (manufactured by Wako Pure Chemical Industries, Ltd.), was prepared. The amount of maltose is the same as the amount of glucose in Example 1.

### [Example 3] Cell-preservation liquid containing mannitol and baking soda

A cell-preservation liquid similar to that of Example 1, except that glucose in Example 1 was replaced by mannitol (manufactured by Wako Pure Chemical Industries, Ltd.), was prepared. The amount of mannitol is the same as the amount of glucose in Example 1.

### [Example 4] Cell-preservation liquid containing sucrose and baking soda

A cell-preservation liquid similar to that of Example 1, except that glucose in Example 1 was replaced by sucrose (manufactured by SIGMA CORPORATION), was prepared. The amount of sucrose is the same as the amount of glucose in Example 1.

### [Example 5] Cell-preservation liquid containing trehalose and baking soda

A cell-preservation liquid similar to that of Example 1, except that glucose in Example 1 was replaced by trehalose (manufactured by Wako Pure Chemical Industries, Ltd.), was prepared. The amount of trehalose is the same as the amount of glucose in Example 1.

### Example 6] Cell-preservation liquid containing no bicarbonate ion and/or no carbonate ion

A cell-preservation liquid similar to that of Example 1, except that bicarbonate ion and/or carbonate ion (baking soda) was not contained, was prepared. Accordingly, the liquid has the following composition.

| | |
|---|---|
| Glucose | 5.5 mM |
| Sodium ion | 142.4 mM |
| Potassium ion | 5.4 mM |
| Bicarbonate ion and/or carbonate ion | 0 mM |
| Calcium ion | 1.8 mM |
| Magnesium ion | 0.8 mM |
| Chloride ion | 132.4 mM |
| Phosphate ion | 1.0 mM |
| Sulfate ion | 0.8 mM |
| Pure water | |
| Total | 1,000 mL |

### [Example 7] Cell-preservation liquid having osmotic pressure of 200 mOsm/kg

A cell-preservation liquid having the following composition was prepared. The osmotic pressure was about 200 mOsm/kg.

| | |
|---|---|
| Glucose | 1,000 mg |
| Calcium chloride | 200 mg |
| Potassium chloride | 400 mg |
| Magnesium sulfate | 98 mg |
| Sodium chloride | 4,000 mg |
| Phosphoric acid hydrogen sodium hydrate | 140 mg |
| Carbonic acid hydrogen sodium (baking soda) | 2,000 mg |
| Pure water | |
| Total | 1,000 mL |

The ion composition of this cell-preservation liquid is shown in the following.

| | |
|---|---|
| Glucose | 5.5 mM |
| Sodium ion | 93.0 mM |
| Potassium ion | 5.4 mM |
| Bicarbonate ion and/or carbonate ion | 23.8 mM |
| Calcium ion | 1.8 mM |
| Magnesium ion | 0.8 mM |
| Chloride ion | 79.6 mM |
| Phosphate ion | 1.0 mM |
| Sulfate ion | 0.8 mM |
| Pure water | |
| Total | 1,000 mL |

### [Example 8] Cell-preservation liquid having osmotic pressure of 240 mOsm/kg

A cell-preservation liquid having an osmotic pressure of about 240 mOsm/kg was prepared. The osmotic pressure was regulated by addition of sodium chloride (1,240 mg) to the composition of Example 7.

### [Example 9] Cell-preservation liquid having osmotic pressure of 300 mOsm/kg

A cell-preservation liquid having an osmotic pressure of about 300 mOsm/kg was prepared. The osmotic pressure was regulated by addition of sodium chloride (3,100 mg) to the composition of Example 7.

### [Example 10] Cell-preservation liquid having osmotic pressure of 400 mOsm/kg

A cell-preservation liquid having an osmotic pressure of about 400 mOsm/kg was prepared. The osmotic pressure was regulated by addition of sodium chloride (6,200 mg) to the composition of Example 7.

### [Example 11] Cell-preservation liquid having osmotic pressure of 500 mOsm/kg

A cell-preservation liquid having an osmotic pressure of about 500 mOsm/kg was prepared. The osmotic pressure was regulated by addition of sodium chloride (9,610 mg) to the composition of Example 7.

### [Example 12] Cell-preservation liquid having pH of 5.0

A cell-preservation liquid having the following composition was prepared. The osmotic pressure and pH of the cell-preservation liquid were about 299 mOsm/kg and about 5.0, respectively.

| | |
|---|---|
| Glucose | 1,000 mg |
| Calcium chloride | 200 mg |
| Potassium chloride | 400 mg |
| Magnesium sulfate | 98 mg |
| Sodium chloride | 6,800 mg |
| Phosphoric acid hydrogen sodium hydrate | 140 mg |
| Carbonic acid hydrogen sodium (baking soda) | 2,000 mg |
| Sodium hydroxide | 1.08 mg |
| Hydrochloric acid | 23.2 mg |
| Pure water | |
| Total | 1,000 mL |

The ion composition of this cell-preservation liquid is shown in the following.

| | |
|---|---|
| Glucose | 5.4 mM |
| Sodium ion | 138.8 mM |
| Potassium ion | 5.2 mM |
| Bicarbonate ion and/or carbonate ion | 23.2 mM |
| Calcium ion | 1.8 mM |
| Magnesium ion | 0.8 mM |
| Chloride ion | 145.0 mM |
| Phosphate ion | 1.0 mM |
| Sulfate ion | 0.8 mM |
| Pure water | |
| Total | 1,000 mL |

### [Example 13] Cell-preservation liquid having pH of 6.0

A cell-preservation liquid having the following composition was prepared. The osmotic pressure and pH of the cell-preservation liquid were about 293 mOsm/kg and about 6.0, respectively.

| | |
|---|---|
| Glucose | 1,000 mg |
| Calcium chloride | 200 mg |
| Potassium chloride | 400 mg |
| Magnesium sulfate | 98 mg |
| Sodium chloride | 6,800 mg |
| Phosphoric acid hydrogen sodium hydrate | 140 mg |
| Carbonic acid hydrogen sodium (baking soda) | 2,000 mg |
| Hydrochloric acid | 14 mg |
| Pure water | |
| Total | 1,000 mL |

The ion composition of this cell-preservation liquid is shown in the following.

| | |
|---|---|
| Glucose | 5.5 mM |
| Sodium ion | 139.2 mM |
| Potassium ion | 5.3 mM |
| Bicarbonate ion and/or carbonate ion | 23.5 mM |
| Calcium ion | 1.8 mM |
| Magnesium ion | 0.8 mM |
| Chloride ion | 137.4 mM |
| Phosphate ion | 1.0 mM |
| Sulfate ion | 0.8 mM |
| Pure water | |
| Total | 1,000 mL |

### [Example 14] Cell-preservation liquid having pH of 7.0

A cell-preservation liquid having the following composition was prepared. The osmotic pressure and pH of the cell-preservation liquid were about 283 mOsm/kg and about 7.0, respectively.

| | |
|---|---|
| Glucose | 1,000 mg |
| Calcium chloride | 200 mg |
| Potassium chloride | 400 mg |
| Magnesium sulfate | 98 mg |
| Sodium chloride | 6,800 mg |
| Phosphoric acid hydrogen sodium hydrate | 140 mg |
| Carbonic acid hydrogen sodium (baking soda) | 2,000 mg |
| Hydrochloric acid | 2.8 mg |
| Pure water | |
| Total | 1,000 mL |

The ion composition of this cell-preservation liquid is shown in the following.

| | |
|---|---|
| Glucose | 5.5 mM |
| Sodium ion | 140.8 mM |
| Potassium ion | 5.4 mM |
| Bicarbonate ion and/or carbonate ion | 23.7 mM |
| Calcium ion | 1.8 mM |
| Magnesium ion | 0.8 mM |
| Chloride ion | 127.8 mM |
| Phosphate ion | 1.0 mM |
| Sulfate ion | 0.8 mM |
| Pure water | |
| Total | 1,000 mL |

### [Example 15] Cell-preservation liquid having pH of 8.0

A cell-preservation liquid having the following composition was prepared. The osmotic pressure and pH of the cell-preservation liquid were about 281 mOsm/kg and about 8.0, respectively.

| | |
|---|---|
| Glucose | 1,000 mg |
| Calcium chloride | 200 mg |
| Potassium chloride | 400 mg |
| Magnesium sulfate | 98 mg |
| Sodium chloride | 6,800 mg |
| Phosphoric acid hydrogen sodium hydrate | 140 mg |
| Carbonic acid hydrogen sodium (baking soda) | 2,000 mg |
| Sodium hydroxide | 0.6 mg |
| Pure water | |
| Total | 1,000 mL |

The ion composition of this cell-preservation liquid is shown in the following.

| | |
|---|---|
| Glucose | 5.5 mM |
| Sodium ion | 141.7 mM |
| Potassium ion | 5.4 mM |
| Bicarbonate ion and/or carbonate ion | 23.8 mM |
| Calcium ion | 1.8 mM |
| Magnesium ion | 0.8 mM |
| Chloride ion | 125.2 mM |
| Phosphate ion | 1.0 mM |
| Sulfate ion | 0.8 mM |
| Pure water | |
| Total | 1,000 mL |

### [Example 16] A cell-preservation liquid for measurement of variations with time of the cell survival rates

A cell-preservation liquid having the following composition was prepared. The osmotic pressure of the cell-preservation liquid was about 288 mOsm/kg.

| | |
|---|---|
| Glucose | 10,000 mg |
| Calcium chloride | 200 mg |
| Potassium chloride | 400 mg |
| Magnesium sulfate | 98 mg |
| Sodium chloride | 6,800 mg |
| Phosphoric acid hydrogen sodium hydrate | 140 mg |
| Carbonic acid hydrogen sodium (baking soda) | 2,000 mg |
| Pure water | |
| Total | 1,000 mL |

The ion composition of this cell-preservation liquid is shown in the following.

| | |
|---|---|
| Glucose | 47.8 mM |
| Sodium ion | 121.4 mM |
| Potassium ion | 4.6 mM |
| Bicarbonate ion and/or carbonate ion | 20.5 mM |
| Calcium ion | 1.6 mM |
| Magnesium ion | 0.7 mM |
| Chloride ion | 110.0 mM |
| Phosphate ion | 0.9 mM |
| Sulfate ion | 0.7 mM |
| Pure water | |
| Total | 1,000 mL |

### [Example 17] A cell-preservation liquid for cell culture after preservation

cell-preservation liquid having the following composition was prepared. The osmotic pressure of the cell-preservation liquid was about 283 mOsm/kg.

| | |
|---|---|
| Glucose | 1,000 mg |
| Calcium chloride | 200 mg |
| Potassium chloride | 400 mg |
| Magnesium sulfate | 98 mg |
| Sodium chloride | 6,800 mg |
| Phosphoric acid hydrogen sodium hydrate | 140 mg |
| Carbonic acid hydrogen sodium (baking soda) | 2,000 mg |
| Hydrochloric acid | 2.8 mg |
| Pure water | |
| Total | 1,000 mL |

The ion composition of this cell-preservation liquid is shown in the following.

| | |
|---|---|
| Glucose | 5.5 mM |
| Sodium ion | 140.8 mM |
| Potassium ion | 5.4 mM |
| Bicarbonate ion and/or carbonate ion | 23.7 mM |
| Calcium ion | 1.8 mM |
| Magnesium ion | 0.8 mM |
| Chloride ion | 127.8 mM |
| Phosphate ion | 1.0 mM |
| Sulfate ion | 0.8 mM |
| Pure water | |
| Total | 1,000 mL |

### [Example 18] A cell-preservation liquid for measurement of variations with time of the cell survival rates

An injection solution 250 ml having the following composition of 1) below (KN-solution, manufactured by Otsuka Pharmaceutical Co., Ltd.) and sodium bicarbonate injection solution as defined in the Japanese Pharmacopoeia 30 mL (SOLITA™-T No.2, manufactured by AJINOMOTO CO., Inc.) were mixed in Ringer's solution as defined in the Japanese Pharmacopoeia 800 mL (Manufactured by Fuso Pharmaceutical Industries, Ltd.) to prepare a cell-preservation liquid.

### 1) Injection solution containing the components described below in 1,000 mL of the solution;

| | |
|---|---|
| Sodium chloride | 1.92 g |
| Potassium chloride | 1.00 g |
| Sodium lactate | 2.80 g |
| Magnesium chloride | 0.10 g |
| Monosodium phosphate | 0.14 g |
| Dipotassium phosphate | 1.00 g |
| Glucose | 23.5 g |

In this case, the final composition was the following 2), and the osmotic pressure and pH were about 317.7 mOsm/kg and about 7.749 (24.4°C), respectively.

### 2) Final composition:

| | |
|---|---|
| Glucose | 30.2 mM |
| Sodium ion | 145.7 mM |
| Potassium ion | 8.7 mM |
| Bicarbonate ion and/or carbonate ion | 23.1 mM |
| Calcium ion | 3.3 mM |
| Magnesium ion | 0.2 mM |
| Chloride ion | 126.4 mM |
| Phosphate ion | 1.6 mM |
| Lactate ion | 5.8 mM |
| Total liquid volume | 1,080 mL |

### [Example 19] A cell-preservation liquid for measurement of variations with time of the cell survival rates

An injection solution 300 ml having the composition of Example 18-1) (KN-solution, manufactured by Otsuka Pharmaceutical Co., Ltd.) and sodium bicarbonate injection solution as defined in the Japanese Pharmacopoeia 30 mL (SOLITA™-T No.2, manufactured by AJINOMOTO CO., Inc.) were mixed in Ringer's solution as defined in the Japanese Pharmacopoeia 800 mL (Manufactured by Fuso Pharmaceutical Industries, Ltd.) to prepare a cell-preservation liquid.
In this case, the final composition was as follows, and the osmotic pressure and pH were about 316.7 mOsm/kg and about 7.351 (25.2°C), respectively.

### Final composition:

| | |
|---|---|
| Glucose | 34.6 mM |
| Sodium ion | 141.9 mM |
| Potassium ion | 9.4 mM |
| Bicarbonate ion and/or carbonate ion | 22.1 mM |
| Calcium ion | 3.2 mM |
| Magnesium ion | 0.3 mM |
| Chloride ion | 122.9 mM |
| Phosphate ion | 1.8 mM |
| Lactate ion | 6.6 mM |
| Total liquid volume | 1,130 mL |

### [Example 20] A cell-preservation liquid for measurement of variations with time of the cell survival rates

An injection solution 400 ml having the following composition of 1) below (KN-solution, manufactured by Otsuka Pharmaceutical Co., Ltd.) and sodium bicarbonate injection solution as defined in the Japanese Pharmacopoeia 30 mL (SOLITA™-T No.2, manufactured by AJINOMOTO CO., Inc.) were mixed in Ringer's solution as defined in the Japanese Pharmacopoeia 800 mL (Manufactured by Fuso Pharmaceutical Industries, Ltd.) to prepare a cell-preservation liquid.

### 1) Injection solution containing the components described below in 1,000 mL of the solution;

| | |
|---|---|
| Sodium chloride | 0.270 w/v% |
| Potassium chloride | 0.149 w/v% |
| Sodium lactate | 0.224 w/v% |
| Sodium dihydrogenphosphate | 0.031 w/v% |
| Sodium monohydrogenphosphate | 0.287 w/v% |
| Glucose | 3.2 w/v% |
| L-lactic acid as an additive | 8 mEq/L |

In this case, the final composition was the following 2), and the osmotic pressure and pH were about 339.7 mOsm/kg and about 7.442 (24.2°C), respectively.

### 2) Final composition:

| | |
|---|---|
| Glucose | 57.8 mM |
| Sodium ion | 143.3 mM |
| Potassium ion | 9.1 mM |
| Bicarbonate ion and/or carbonate ion | 20.3 mM |
| Calcium ion | 2.9 mM |
| Magnesium ion | 0.0 mM |
| Chloride ion | 122.7 mM |
| Phosphate ion | 3.3 mM |
| Lactate ion | 9.1 mM |
| Pure water | |
| Total | 1,230 mL |

### [Example 21] A cell-preservation liquid for measurement of variations with time of the cell survival rates

An injection solution 450 ml having the following composition of 1) below (KN-solution, manufactured by Otsuka Pharmaceutical Co., Ltd.) and sodium bicarbonate injection solution as defined in the Japanese Pharmacopoeia 30 mL (SOLITA™-T No.2, manufactured by AJINOMOTO CO., Inc.) were mixed in Ringer's solution as defined in the Japanese Pharmacopoeia 800 mL (Manufactured by Fuso Pharmaceutical Industries, Ltd.) to prepare a cell-preservation liquid.
In this case, the final composition was as follows, and the osmotic pressure and pH were about 316.7 mOsm/kg and about 7.351 (25.2°C), respectively.

### Final composition:

| | |
|---|---|
| Glucose | 62.4 mM |
| Sodium ion | 141.0 mM |
| Potassium ion | 9.5 mM |
| Bicarbonate ion and/or carbonate ion | 19.5 mM |
| Calcium ion | 2.8 mM |
| Magnesium ion | 0.0 mM |
| Chloride ion | 120.5 mM |
| Phosphate ion | 3.5 mM |
| Lactate ion | 9.8 mM |
| Pure water | |
| Total | 1,280 mL |

### [Comparative Example 1] Cell-preservation liquid containing no saccharide

A cell-preservation liquid similar to that of Example 1 except that glucose was not contained was prepared. Accordingly, the liquid has the following composition.

| | |
|---|---|
| Glucose | 0 mM |
| Sodium ion | 145.3 mM |
| Potassium ion | 5.4 mM |
| Bicarbonate ion and/or carbonate ion | 23.7 mM |
| Calcium ion | 1.8 mM |
| Magnesium ion | 0.8 mM |
| Chloride ion | 132.4 mM |
| Phosphate ion | 1.0 mM |
| Sulfate ion | 0.8 mM |
| Pure water | |
| Total | 1,000 mL |

### [Comparative Example 2] Phosphate buffer

Phosphate buffer to be generally used as a cell-preservation liquid was used as a cell-preservation liquid.

### [Comparative Example 3] Physiological saline

Physiological saline to be generally used as a cell-preservation liquid (containing 2.5 v/v% human albumin) was used as a cell-preservation liquid.

### [Comparative Example 4] Euro-Collins solution

Euro-Collins solution that is generally commercially available as an organ-preservation liquid was used as a cell-preservation liquid. In the composition of Euro-Collins solution to be used in the present Comparative Example, the saccharide content, sodium ion concentration, and potassium ion concentration were 194.3 mM (glucose), 10.0 mM, and 115.0 mM, respectively.

### [Comparative Example 5] Cell-preservation liquid having pH of 9.0

A cell-preservation liquid having the following composition was prepared. The osmotic pressure and pH of the cell-preservation liquid were about 285 mOsm/kg and about 9.0, respectively.

| | |
|---|---|
| Glucose | 1,000 mg |
| Calcium chloride | 200 mg |
| Potassium chloride | 400 mg |
| Magnesium sulfate | 98 mg |
| Sodium chloride | 6,988 mg |
| Phosphoric acid hydrogen sodium hydrate | 140 mg |
| Carbonic acid hydrogen sodium (baking soda) | 2,000 mg |
| Sodium hydroxide | 3.2 mg |
| Pure water | |
| Total | 1,000 mL |

The ion composition of this cell-preservation liquid is shown in the following.

| | |
|---|---|
| Glucose | 5.5 mM |
| Sodium ion | 147.1 mM |
| Potassium ion | 5.3 mM |
| Bicarbonate ion and/or carbonate ion | 23.7 mM |
| Calcium ion | 1.8 mM |
| Magnesium ion | 0.8 mM |
| Chloride ion | 128.1 mM |
| Phosphate ion | 1.0 mM |
| Sulfate ion | 0.8 mM |
| Pure water | |
| Total | 1,000 mL |

### [Experimental Example 1] Cell survival rate in cell-preservation liquid of the present invention

A comparison of the cell-preservation abilities of cell-preservation liquids containing various saccharides and a comparison thereof with conventional cell-preservation liquids were performed under the condition where the osmotic pressures of the preservation liquids were regulated to the same level. Specifically, the concentrations of cell suspensions of a lymphocyte (T-lymphocyte) were regulated to about 2.0 x 10⁶ cells/mL using the cell-preservation liquids of Examples 1 to 6 and Comparative Examples 1 to 4, and the suspensions were preserved in the respective preservation liquids under a cold condition (4°C). After 4 days of preservation, the survival rates of the lymphocyte were calculated from a determination of living and dead cells performed by trypan blue staining (trypan blue exclusion). The results are shown in Table 1.

**[Table 1]**

| | Cell-survival rate (%) |
|---|---|
| Example 1 | 73.6 |
| Example 2 | 67.9 |
| Example 3 | 39.8 |
| Example 4 | 35.3 |
| Example 5 | 34.0 |
| Example 6 | 48.5 |
| Comparative Example 1 | 24.0 |
| Comparative Example 2 | 26.9 |
| Comparative Example 3 | 11.9 |
| Comparative Example 4 | * |

| | |
|---|---|
| * Not measurable due to occurrence of aggregates | |

As is clear from the results shown in Table 1, the cell-preservation liquids each containing a saccharide and bicarbonate ion and/or carbonate ion as active ingredients (Examples 1 to 6) were found to have higher survival rates than those of Comparative Examples. Of those, the preservation liquids of Examples 1 and 2 were found to have particularly high survival rates.

### [Experimental Example 2] Effect of osmotic pressure of cell-preservation liquid

Osmotic pressure dependencies of preservation liquids were examined. Specifically, the concentrations of cell suspensions of a lymphocyte (T-lymphocyte) were regulated to about 1.5 x 10⁶ cells/mL using the cell-preservation liquids of Examples 7 to 11, and the suspensions were preserved in the respective preservation liquids under a cold condition (4°C). After 4 days of preservation, the survival rates of the lymphocyte were calculated from a determination of living and dead cells performed by trypan blue staining. The results are shown in Table 2. A comparison was performed with commercially available cell-preservation liquids (Comparative Examples 2 and 3).

**[Table 2]**

| | Cell-survival rate (%) |
|---|---|
| Example 8 | 60.4 |
| Example 9 | 59.1 |
| Example 10 | 42.7 |
| Example 11 | 40.5 |
| Comparative Example 2 | 26.9 |
| Comparative Example 3 | 11.9 |

From the results shown in Table 2, the cell-preservation liquids each having a saccharide, potassium ion, and sodium ion as active ingredients and having any osmotic pressure in the range of 200 to 500 mOsm/kg (Examples 7 to 11) were found to have higher cell-survival rates than the commercially available cell-preservation liquids (Comparative Examples 2 and 3), and the effectiveness of the present invention was proved. In particular, the cell-preservation liquids having an osmotic pressure in the range of 200 to 300 mOsm/kg (Examples 7 to 9) were found to have even higher survival rates.

### [Experimental Example 3] Effect of pH of cell-preservation liquid

pH dependencies were examined under the conditions where glucose, calcium ion, and sodium ion were present in a cell-preservation liquid and the osmotic pressures were regulated to approximately the same level. Specifically, the concentrations of cell suspensions of a lymphocyte (T-lymphocyte) were regulated to about 1.0 x 10⁶ cells/mL using the cell-preservation liquids of Examples 12 to 15 and Comparative Example 5, and the suspensions were preserved in the respective preservation liquids under a cold condition (4°C). After 4 days of the preservation, the survival rates of the lymphocyte were calculated from a determination of living and dead cells performed by trypan blue staining. The results are shown in Table 3.

**[Table 3]**

| | Cell-survival rate (%) |
|---|---|
| Example 12 | 36.8 |
| Example 13 | 52.1 |
| Example 14 | 51.6 |
| Example 15 | 57.6 |
| Comparative Example 5 | * |

| | |
|---|---|
| * Not measurable due to occurrence of aggregates | |

As is clear form the results shown in Table 3, the survival rates were found to be relatively high in the case of the cell-preservation liquids having pH values in the range of 5.0 to 8.0 (Examples 12 to 15), while the survival rates were found to be particularly high in the case of the cell-preservation liquids having pH values in the range of pH 6.0 to 8.0 (Examples 13 to 15).

### [Experimental Example 4] Variation with time of cell survival rate

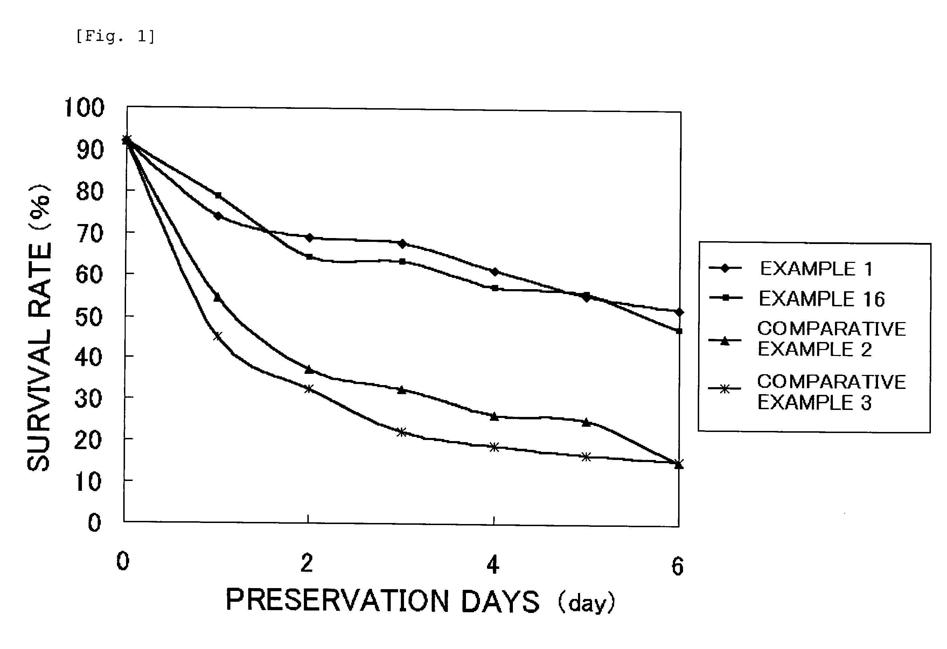

Fig. 1 shows the results obtained by measurement of variations with time of the cell survival rates of the cell-preservation liquids of Examples 1 and 16 and of conventional cell-preservation liquids (Comparative Examples 2 and 3). The cell-preservation liquids of the present invention were found to have the relatively high cell survival rates even after 6 days of beginning of the preservation.

### [Experimental Example 5] Cell culture after preservation

The concentration of a lymphocyte in the logarithmic growth phase obtained after 5 days of beginning of activated culture was regulated to 20 x 10⁶ cells/mL using the cell-preservation liquid of Example 17, and the suspensions were preserved under a cold condition for 4 days. After the preservation, the cells were collected, and an aliquot thereof was added to a medium for lymphocytes (ALyS505N medium, manufactured by Cell Science & Technology Institute, Inc.) containing 10% FBS without a washing operation, followed by culture at an inoculation concentration of 2.7 x 10⁵ cells/mL in a CO₂ atmosphere at 37°C for 4 days. As a result, the lymphocyte concentration was increased to 1.7 x 10⁶ cells/mL after 4 days. Accordingly, the cell-preservation liquid of Example 17 was confirmed not to inhibit cell culture.

### [Experimental Example 6] Variation,with time of cell survival rate

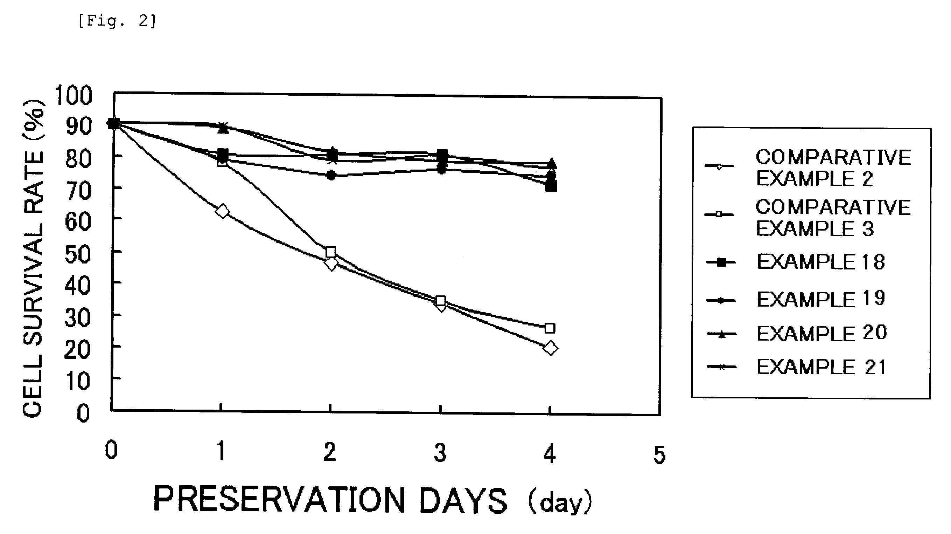

Fig. 2 shows the results obtained by measurement of variations with time of the cell survival rates of the cell-preservation liquids of Examples 18 and 21 and conventional cell-preservation liquids (Comparative Examples 2 and 3). The cell-preservation liquids of the present invention were found to have relatively high cell survival rates even after 4 days of beginning of the preservation.

As described above, use of the cell-preservation liquid of the present invention enables improvement of the cell-preservation ability not only in the case of cryopreservation but also under a cold condition, resulting in increase of the cell survival rate. In addition, the present invention provides a cell-preservation method under a cold condition, so that cell species that are difficult to preserve may be preserved. Moreover, even in the case that an expensive apparatus/equipment such as a programming freezer that is required for cryopreservation cannot be provided, cells may be preserved. Furthermore, when the cell-preservation liquid of the present invention is used, cell culture may be performed by inoculating cells in a medium with the cells are suspended in the preservation liquid without a washing step or the like, so that the cells may be easily cultured. For example, the cell-preservation liquid of the present invention may be used in the field of immunocyte therapy, which is becoming popular, when a lymphocyte obtained by activated culture is transported from a facility for cell culture service to a patient. The preservation liquid is considered to suppress a decrease in the cell survival rate, maintain cell quality, and be consequently useful for application to treatment.

## Claims

1. A cell-preservation liquid **characterized by** comprising at least a saccharide, sodium ion, and potassium ion, wherein the molar concentration of the contained sodium ion is equal to or more than that of the potassium ion.

2. The cell-preservation liquid according to Claim 1, wherein the molar ratio of the potassium ion and the sodium ion is 1:1 to 1:85.

3. The cell-preservation liquid according to Claim 1, wherein the saccharide content is 0.5 to 150 mM.

4. The cell-preservation liquid according to Claim 1, wherein the saccharide is one or a plurality of saccharides selected from the group consisting of glucose, maltose, mannitol, sucrose, and trehalose.

5. The cell-preservation liquid according to Claim 1, wherein the saccharide is glucose and/or maltose.

6. The cell-preservation liquid according to Claim 1, which further comprises bicarbonate ion and/or carbonate ion.

7. The cell-preservation liquid according to Claim 6, wherein the bicarbonate ion and/or carbonate ion content is 1 to 75 mM.

8. The cell-preservation liquid according to Claim 1, which further comprises any one kind or a plurality of ions selected from the group consisting of calcium ion, magnesium ion, chloride ion, phosphate ion, sulfate ion, and lactate ion.

9. The cell-preservation liquid according to Claim 8, which comprises the following composition in 1,000 mL of a solution:
| | |
|---|---|
| Saccharide | 0.5 to 150 mM |
| Sodium ion | 20 to 260 mM |
| Potassium ion | 3 to 125 mM |
| Bicarbonate ion and/or carbonate ion | 1 to 75 mM |
| Calcium ion | 1 to 4 mM |
| Magnesium ion | 0 to 1 mM |
| Chloride ion | 75 to 245 mM |
| Phosphate ion | 0.5 to 65 mM |
| Sulfate ion | 0 to 1 mM |
| Lactate ion | 0 to 20 mM. |

10. A method for preparing the cell-preservation liquid according to claim 1, which is **characterized in that** a sodium salt, a potassium salt and a saccharide are mixed with a solvent selected from the group consisting of a dehydration supplementary liquid and pure water such that the molar concentration of the contained sodium ion is equal to or more than that of the potassium ion.

11. The method for preparing the cell-preservation liquid according to Claim 10, which is **characterized** defined as in that Ringer's solution defined as in the Japanese Pharmacopoeia, sodium bicarbonate injection in the Japanese Pharmacopoeia, and a commercially available dehydration supplementary liquid are mixed at a volume ratio of 80:3:20 to 50.

12. The method for preparing the cell-preservation liquid according to Claim 11, wherein the commercially available dehydration supplementary liquid is selected from the injections shown in 1) and 2) below:
1) an injection containing the components described below in 1,000 mL of the solution:
| | |
|---|---|
| Sodium chloride | 1.92 g |
| Potassium chloride | 1.00 g |
| Sodium lactate | 2.80 g |
| Magnesium chloride | 0.10 g |
| Monosodium phosphate | 0.14 g |
| Dipotassium phosphate | 1.00 g |
| Glucose | 23.5 g*i* |
2) an injection comprising the composition described below:
| | |
|---|---|
| Sodium chloride | 0.270 w/v% |
| Potassium chloride | 0.149 w/v% |
| Sodium lactate | 0.224 w/v% |
| Sodium dihydrogenphosphate | 0.031 w/v% |
| Sodium monohydrogenphosphate | 0.287 w/v% |
| Glucose | 3.2 w/v% |
| L-lactic acid as an additive | 8 mEq/L. |

13. A cell-preservation method **characterized in that** a cell is preserved under a temperature of above freezing to 25 using the cell-preservation liquid according to Claims 1 to 9.

14. The cell-preservation method according to Claim 13, wherein the cell is a lymphocyte.

15. A cell-culture method comprising: suspending a cell in the cell-preservation liquid according to Claims 1 to 9; and inoculating the suspension as is in a medium to culture the cell.
